**Europäisches Patentamt**

**European Patent Office**

**Office européen des brevets**

(19)

(11) Veröffentlichungsnummer: **0 391 253**

**A1**

# (12) EUROPÄISCHE PATENTANMELDUNG

(21) Anmeldenummer: **90105993.1**

(22) Anmeldetag: **29.03.90**

(51) Int. Cl.5: **C12N 9/00, C12N 9/56**

(30) Priorität: **06.04.89 DE 3911099**

(43) Veröffentlichungstag der Anmeldung:
**10.10.90 Patentblatt 90/41**

(84) Benannte Vertragsstaaten:
**GR**

(71) Anmelder: **Henkel Kommanditgesellschaft auf Aktien**
**Henkelstrasse 67**
**D-4000 Düsseldorf 13(DE)**

Anmelder: **BIOCHEMIE Gesellschaft m.b.H.**

**A-6250 Kundl Tirol(AT)**

(72) Erfinder: **Wüst, Willi, Dr.**
**Fasanenring 32**
**D-4030 Ratingen(DE)**
Erfinder: **Kühne, Norbert**
**Dürerstrasse 63**
**D-5657 Haan 1(DE)**
Erfinder: **Palma, Norbert, Dr.**
**Kleinsöll 127**
**A-6250 Breitenbach(AT)**

(54) **Verfahren zur Reinigung von Enzymlösungen.**

(57) Bei einem Verfahren zur Reinigung und Desodorierung wäßriger, durch Fermentation von Bakterien oder Pilzen gewonnener Enzymlösungen sollte die Enzymausbeute und die Filtrierbarkeit der abzutrennenden Niederschläge verbessert werden. Dies gelang dadurch, daß man die Enzymlösung mit mindestens einem Maskierungsmittel, ausgewählt aus der Gruppe
- Säuren des Bors und/oder deren wasserlöslichen Salze
- schwefelige Säure und/oder deren wasserlöslichen Salze
- Hydroxycarbonsäuren und/oder deren wasserlöslichen Salze
- mehrfunktionellen Alkoholen
versetzt und hernach eine Fällung erzeugt, in dem man in beliebiger Reihenfolge zwei wasserlösliche, sich gegenseitig fällende ionische Verbindungen zugibt, gewünschtenfalls mit weiteren Adsorptionsmitteln versetzt und anschließend die Feststoffe von der Enzymlösung abtrennt.

## Verfahren zur Reinigung Von Enzymlösungen

Die Erfindung betrifft ein Verfahren zur Reinigung, insbesondere Desodorierung und Entfärbung von Enzymlösungen, wie sie durch Fermentation von Bakterien oder Pilzen entstehen.

Zahlreiche Enzyme, insbesondere Hydrolasen wie beispielsweise Proteasen, Amylasen oder Lipasen, werden durch Fermentation von Mikroorganismen hergestellt. Geeignete Mikroorganismen und Herstellverfahren sind beispielsweise in den folgenden Patenten bzw. Patentanmeldungen beschrieben: DE 18 00 508, DE 22 24 777, DE 25 51 742, US 3 827 938, WO 88/01293, DE 18 07 185, US 3 740 318, DE 23 34 463, DE 20 26 092, EP 0 232 169, EP 0 220 921, EP 0 247 647 und EP 0 246 678.

Bei der Fermentation der Stämme werden nach Abtrennen der Biomasse Enzymlösungen erhalten, die Farb- und Geruchsstoffe als Beimengungen aufweisen.

Für zahlreiche Anwendungen, z.B. für den Einsatz der Enzymlösungen in Flüssigwaschmitteln sind derartige Beimengungen nicht tolerierbar. Bei der technischen Herstellung der Enzyme versucht man daher die Verunreinigungen durch Fällungsverfahren abzutrennen. Bisher bekannt gewordene Fällungsverfahren haben jedoch den Nachteil, daß zur Erzielung einer guten Farbqualität erhebliche Ausbeuteverluste in Kauf genommen werden müssen. Soweit zur Fällung die an sich hier günstigen Bentonite verwendet werden, treten zudem Filtrationsschwierigkeiten auf.

Auf der anderen Seite ist es bekannt, Enzymlösungen gewisse stabilisierende Zusätze beizugeben. Es wird angenommen, daß diese Zusätze Hydrolasen zu maskieren vermögen, und dadurch den Selbstangriff, z.B. bei Proteasen, verhindern. Derartige bekannte Maskierungsmittel sind Borsäure und ihre Salze, Natriumsulfit, mehrfunktionelle Garbonsäuren, Hydroxycarbonsäuren oder auch Glykole oder Glycerin. Bekannt sind Maskierungsmittel beispielsweise aus DE 31 25 533, EP 0 080 223, EP 0 080 748, EP 0 126 508, DE 34 18 295.

Vor dem Hintergrund dieses Standes der Technik ist es Aufgabe der Erfindung) ein Verfahren bereitzustellen, das es erlaubt, Enzymlösungen von störenden farblichen und geruchlichen Beimengungen unter Minimierung der Ausbeuteverluste zu reinigen und dabei Ausfällungen zu erhalten, die leicht abtrennbar sind.

Gegenstand der Erfindung ist somit ein Verfahren zur Reinigung und Desodorierung wäßriger, durch Fermentation von Bakterien oder Pilzen gewonnener Enzymlösungen, durch in situ Herstellung eines wasserunlöslichen Niederschlags, Adsorption von nicht gewünschten Beimengen an diesem Niederschlag und anschließende Fest-Flüssig-Trennung, dadurch gekennzeichnet, daß man die Enzymlösungen mit mindestens einem Maskierungsmittel, ausgewählt aus der Gruppe

- Säuren des Bors und/oder deren wasserlöslichen Salze
- schwefelige Säure und/oder deren wasserlöslichen Salze
- Hydroxycarbonsäuren und/oder deren wasserlöslichen Salze
- mehrfunktionellen Alkoholen

versetzt und hernach eine Fällung erzeugt, in dem man in beliebiger Reihenfolge zwei wasserlösliche, sich gegenseitig fällende ionische Verbindungen zugibt, gewünschtenfalls mit weiteren Adsorptionsmitteln versetzt und anschließend die Feststoffe von der Enzymlösung abtrennt.

In einer ganz allgemeinen Form besteht das erfindungsgemäße Verfahren darin, einer Enzymlösung, wie sie durch Fermentation erhalten und nur durch Abtrennung der Biomasse vorgereinigt wurde, bereits ein Maskierungsmittel beizufügen und dann durch Fällung weiterzureinigen.

Als Maskierungsmittel können zum einen Säuren des Bors und schwefelige Säuren sowie deren Alkalimetallsalze zugesetzt werden. Die zuzusetzenden Mengen betragen 0,5 bis 2 Gew.-%, bezogen auf Enzymlösungen, wobei sich größere Mengen in erster Linie aus wirtschaftlichen Gesichtspunkten verbieten. Geeignete Säuren des Bors sind die Borsäure, die Metaborsäure und/oder die Pentaborsäure. Geeignete Alkalimetallsalze sind daher insbesondere Natriumborat, Natriummetaborat, Borax oder Natriumpentaborat. Weiterhin geeignet ist das Natriumsulfit.

Als weitere Maskierungsmittel können zusammen mit den vorgenannten oder anstelle derselben Dicarbonsäuren und/oder Hydroxycarbonsäuren mit 3 bis 10 C-Atomen eingesetzt werden. Bevorzugt sind die Hydroxydicarbonsäuren, insbesondere Zitronensäure, Weinsäure und deren Isomere. Die Zusatzmenge beträgt 1 bis 5 Gew.-%. Auch hier verbieten sich höhere Zusatzmengen, in erster Linie aus wirtschaftlichen Gesichtspunkten und nicht wegen Nachlassen der technischen Effekte.

Als weitere Maskierungsmittel können zusammen mit den vorgenannten oder anstelle derselben di- und/oder trifunktionelle Alkohole mit 2 bis 10 C-Atomen eingesetzt werden. Diese Alkohole werden in Mengen von 5 bis 30 Gew.-%, insbesondere 10 bis 25 Gew.-%, bezogen auf Enzymlösung, eingesetzt. Bevorzugte Alkohole sind Ethylenglykol, Propylenglykol, Butandiol und Glycerin. Eingesetzt werden können jedoch auch die Di- und/oder Trimeren derselben, also Di- und Triethylenglykol bzw. Di- und Tripropylenglykol. Weiter eingesetzt werden kann auch Trimethylolpropan oder Neopentylglykol.

Nach einer bevorzugten Ausführungsform des erfindungsgemäßen Verfahrens wird in der Enzymlösung eine Fällung erzeugt, in dem man ein wasserlösliches Calciumsalz mit einem Alkalimetallsalz einer Phosphorsäure reagieren läßt. Insbesondere wird das Verhältnis des Calciumsalzes zum Verhältnis des Salzes der Phosphorsäure so gewählt, daß das Molverhältnis Calcium zu Phosphor zwischen 1,7 und 2,5 : 1 beträgt. Dabei hat sich herausgestellt, daß unter diesen Bedingungen Calciumphosphate mit überwiegend amorpher Struktur und großer Oberfläche entstehen, die für die hier zu fällenden farblichen Verunreinigungen günstige Adsorptionsmittel darstellen. Die Fällungsmittelmenge, bezogen auf Enzymlösung, beträgt üblicherweise 0,5 bis 20 Gew.-%.

Zusätzlich zu dieser Maßnahme können gewünschtenfalls auch weitere Adsorptionsmittel der Enzymlösung beigegeben werden. So kann es gewünscht sein, als weitere Adsorptionsmittel Aktivkohle einzusetzen.

Nach einer weiteren Ausführungsform der Erfindung kann es auch gewünscht sein, als zusätzliche Adsorptionsmittel Schichtsilikate einzusetzen, insbesondere Bentonite. Werden Bentonite als Adsorptionsmittel mitverwendet, so ist es bevorzugt, Säure-aktivierte Bentonite einzusetzen. Bekanntlich sind Bentonite, insbesondere im sauren Bereich durch Filtration schlecht abzutrennende Feststoffe.

Im Zusammenhang mit der Erfindung hat sich jedoch gezeigt, daß die hier abzutrennenden Festkörper aufgrund ihrer Wechselwirkung leicht filtrierbar sind. Ein besonders bevorzugter Säure-aktivierter Bentonit hat eine Montmorillonit-Kennzahl von 70 und eine Feinheit von 93 % kleiner 100 $\mu$m. Die Menge an Schichtsilikaten und/oder Aktivkohle beträgt typischerweise 1 bis 10 Gew.-%, bezogen auf Enzymlösung.

Das erfindungsgemäße Verfahren eignet sich zum Desodorieren von Hydrolaselösungen, insbesondere von Lösungen von Proteasen, Amylasen oder Lipasen. Bei den Proteaselösungen sind die Lösungen alkalischer Proteasen bevorzugt, so z.B. die Lösungen von Subtilisin Proteasen, wie Subtilisin Carlsberg oder Subtilisin BPN'. Dabei weisen die zu behandelnden Enzymlösungen Feststoffgehalte von 2 bis 40 Gew.-% auf und enthalten mehr als 10.000, vorzugsweise mehr als 50.000 und insbesondere mehr als 100.000 proteolytische Einheiten/g.

In dem erfindungsgemäßen Verfahren werden bevorzugt solche Enzymlösungen eingesetzt, die durch Abtrennen der bei der Fermentation erzeugten Mikroorganismen und gewünschtenfalls durch weiteres Abtrennen von Salzen und gegebenenfalls durch Eindampfen oder Eindicken mit Hilfe von Membranverfahren erhalten worden sind. Bevorzugt werden Enzymlösungen gereinigt, wie sie nach den Verfahren der DE 35 15 650 oder der DE 37 30 868 erhalten worden sind.

Die Vorteile des erfindungsgemäßen Verfahrens liegen in der kostengünstigen Abtrennung von Farb- und Geruchsstoffen ohne wesentliche Ausbeuteeinbußen und bei der guten Filtrierbarkeit der Niederschläge.

## Beispiele

Durch Fermentation von Bacillus licheniformis gemäß deutscher Patentschrift DE 29 25 427 wurde eine Proteaselösung hergestellt, und diese gemäß der deutschen Patentanmeldung DE 37 30 868 aufkonzentriert. Die so erhaltene Enzymlösung enthielt 200.000 Protease-Einheiten/g.

3,625 t der Lösung wurden mit 0,037 t Borsäure versetzt und 10 min. bei 20°C gerührt. Es wurden sodann 1,8 t Wasser zugegeben und auf 30°C aufgeheizt. Während 30 min. wurden dann 0,711 t Calciumchlorid (mit 2 mol Kristallwasser) als 35 %ige Lösung hinzugegeben (insgesamt 2,03 t Lösung).

Daraufhin wurden 1,564 t einer 33 % eigen Lösung von 0,516 t $Na_2HPO_4 \times 2H_2O$ in Wasser während 70 min. zugegeben und die Temperatur auf 35°C erhöht. Danach ließ man 30 min. bei dieser Temperatur nachrühren. Während der Fällung sank der pH-Wert, so daß dieser mit 0,15 t 50 %iger NaOH auf 6 nachgeregelt werden mußte. Während 5 min. erfolgte dann die Zugabe von 0,15 t eines Säure-aktivierten Bentonits (Bentonit 5 650 der FRINGS-Werke, Montmorillonit-Kennzahl 70, Feinheit 93 % kleiner 100 $\mu$m). Es wurden weitere 30 min. nachgerührt. Schließlich wurden während 5 min. 0,15 t Aktivkohle in 0,45 t Wasser zugegeben und nochmals 30 min. nachgerührt. Die festen Niederschläge wurden anschließend mit Hilfe einer Membranfilterpresse oder einer Stülpfilterzentrifuge abgetrennt. Es wurde eine geruchlich und farblich günstig zu beurteilende Enzymlösung erhalten. Der Erhaltungsgrad des Enzyms betrug 90 - 95 %.

## Vergleichsbeispiel

Das Verfahren des Beispiels wurde wiederholt, jedoch wurde auf die Zugabe der Borsäure verzichtet. Zum Erhalt gleicher Farbqualitäten mußte die Menge an Calciumchlorid und Natriumhydrogenphosphat gesteigert werden. Der Enzymverlust bei gleicher farblicher Qualität der Enzymlösung be-

trug mehr als 20 %.

## Ansprüche

1. Verfahren zur Reinigung und Desodorierung wäßriger durch Fermentation von Bakterien oder Pilzen gewonnener Enzymlösungen, durch in situ Herstellung eines wasserunlöslichen Niederschlags, Adsorption von nicht gewünschten Beimengen an diesem Niederschlag und anschließende Fest-Flüssig-Trennung, dadurch gekennzeichnet, daß man die Enzymlösungen mit mindestens einem Maskierungsmittel, ausgewählt aus der Gruppe
- Säuren des Bors und/oder deren wasserlöslichen Salze
- schwefelige Säure und/oder deren wasserlöslichen Salze
- Hydroxycarbonsäuren und/oder deren wasserlöslichen Salze
- mehrfunktionellen Alkoholen
versetzt und hernach eine Fällung erzeugt, in dem man in beliebiger Reihenfolge zwei wasserlösliche, sich gegenseitig fällende ionische Verbindungen zugibt, gewünschtenfalls mit weiteren Adsorptionsmitteln versetzt und anschließend die Feststoffe von der Enzymlösung abtrennt.

2. Verfahren nach Anspruch 1, dadurch gekennzeichnet, daß man als wasserlösliche, sich gegenseitig fällende Verbindungen, wasserlösliche Calciumsalze und Alkalimetallsalze der Phosphorsäure einsetzt.

3. Verfahren nach Anspruch 1, dadurch gekennzeichnet, daß man als Maskierungsmittel Borsäure, Metaborsäure, Pentaborsäure, schwefelige Säure und/oder deren Alkalimetallsalze in Mengen von 0,5 bis 2 Gew.-%, bezogen auf Enzymlösung, einsetzt.

4. Verfahren nach Anspruch 1, dadurch gekennzeichnet, daß man als Maskierungsmittel Dicarbonsäuren und/oder Hydroxycarbonsäuren mit 3 bis 10 C-Atomen, insbesondere Zitronensäure oder Weinsäure in Mengen von 1 bis 5 Gew.-%, bezogen auf Enzymlösung, einsetzt.

5. Verfahren nach Anspruch 1, dadurch gekennzeichnet, daß man als Maskierungsmittel di- oder trifunktionelle Alkohole mit 2 bis 10 C-Atomen einsetzt.

6. Verfahren nach einem der Ansprüche 1 bis 5, dadurch gekennzeichnet, daß man als Maskierungsmittel Ethylenglykol, Propylenglykol, Butandiol und/oder deren Trimere bzw. Pentamere und/oder Glycerin einsetzt.

7. Verfahren nach einem der Ansprüche 1 bis 6, dadurch gekennzeichnet, daß man zur Erzeugung eines unlöslichen Calciumphosphats die Molverhältnisse wasserlösliches Calciumsalz zu Alkalimetallphosphat, berechnet als Monophosphat, im Bereich von 1,7 1 bis 2,5 : 1 einstellt.

8. Verfahren nach einem der Ansprüche 1 bis 7, dadurch gekennzeichnet, daß man als weitere Adsorptionsmittel Schichtsilikate und/oder Aktivkohle einsetzt.

9. Verfahren nach den Ansprüchen 1 und 8, dadurch gekennzeichnet, daß man als Schichtsilikate Bentonite, insbesondere Säure-aktivierte Bentonite einsetzt.

10. Verfahren nach einem der Ansprüche 1 bis 9, dadurch gekennzeichnet, daß man von Enzymlösungen ausgeht, die zumindest überwiegend Hydrolasen, vorzugsweise Proteasen, Amylasen und/oder Lipasen enthalten.

11. Verfahren nach den Ansprüchen 1 und 10, dadurch gekennzeichnet, daß man von Enzymlösungen ausgeht, die als Proteasen alkalische Proteasen, insbesondere alkalische Proteasen vom Subtilisin-Typ enthalten.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | GB-A-2 178 055 (COLGATE-PALMOLIVE CO.) * Zusammenfassung; Seite 1, Zeilen 51-55 * | 1-11 | C 12 N 9/00 C 12 N 9/56 |
| | --- | | |
| Y,D | EP-A-0 080 223 (UNILEVER NV) * Zusammenfassung * | 1-11 | |
| | --- | | |
| Y | US-A-3 795 586 (JACK ZIFFER) * Spalte 2, Zeilen 23-57 * | 1-11 | |
| | --- | | |
| Y | FR-B- 762 569 (I.G. FARBENINDUSTRIE AG) * Ansprüche * | 1-11 | |
| | --- | | |
| Y | CHEMICAL ABSTRACTS, Band 91, Nr. 2, 9. Juli 1979, Seite 96, Zusammenfassung Nr. 6803q, Columbus, Ohio, US; & JP-A-79 24 907 (NEOS CO., LTD, SHIRAISHI CHUO KENKYUSHO K.K.) 26-07-1977 * Insgesamt * | 1-11 | **RECHERCHIERTE SACHGEBIETE (Int. Cl.5)** C 12 N C 11 D C 07 K |

Der vorliegende Recherchenbericht wurde für alle Patentansprüche erstellt

| Recherchenort | Abschlußdatum der Recherche | Prüfer |
|---|---|---|
| DEN HAAG | 07-06-1990 | ALVAREZ Y ALVAREZ C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument